# EUROPEAN PATENT SPECIFICATION

(11) **EP 3 281 611 B1**
(45) Date of publication and mention of the grant of the patent: **15.01.2020**
(21) Application number: 16725875.5
(22) Date of filing: 07.04.2016
(51) Int. Cl.: A61F 5/453, A61F 5/44

(54) **URINE COLLECTOR DEVICE**
URINSAMMLERVORRICHTUNG
DISPOSITIF DE COLLECTE D'URINE

(30) Priority: 08.04.2015 ES 201530397 U
(43) Date of publication of application: 14.02.2018
(73) Proprietor: García Gómez, Alicia, 03570 Villajoyosa (Alicante) (ES)
(72) Inventor: García Gómez, Alicia, 03570 Villajoyosa (Alicante) (ES)
(74) Representative: Ibarra Garcia, Isabel
(86) International application number: PCT/ES2016/070234
(87) International publication number: WO 2016/162585

(56) References cited:
- GB-A- 994 274
- GB-A- 2 206 050
- US-A- 3 526 227
- US-A- 4 673 401
- US-A1- 2012 165 768

## Description

### TECHNICAL FIELD

The field of application of the present invention is comprised within the sector of the industry intended for manufacturing urine collector devices for sanitary use by male patients with incontinence and for bedridden patients.

More particularly, the object of the invention relates to a device the purpose of which is to provide urine collection means to prevent the use of bedpans or bottles or a urethral catheter in male patients, for which it comprises a receptacle which is coupled, at one end and through several coupling parts, to the user's body in a leak-tight manner, with means for securing it to the patients' waist, and has at the opposite end an outlet which can be connected to a urine collection bag through a draining tube with a nozzle.

### PRIOR ART

Bedpan- or bottle-type urine collector devices the use of which entails various drawbacks are known today as a solution to the problems faced by male patients with urinary incontinence or who are bedridden.

Specifically, in addition to requiring help from another person, these devices are unpleasant for the patient who finds it difficult to urinate, more often than not due to the simple psychological aspects relating to the lack of privacy since, as mentioned, another person is required to place the container and to remove it; likewise, these devices are also very unpleasant for the sick and caregivers who must place them, remove them once used, take them away and clean them after each use, and for a very significant number of times throughout the night, with the subsequent inconvenience for the caregiver, the patient himself and other patients who may share the same room as is often the case in hospitals.

Spanish utility model application number U201130942, published as ES1075847 U, describes an urine collector device for use by men comprising a first receptacle intended for housing the penis and collecting urine, and a second receptacle connected to the first receptacle, as well as respective first and second fixing means for fixing the first receptacle and the second receptacle, where the first fixing means incorporate a first band housed on the perimeter of the end of the first receptacle and a first tape which is a prolongation of the band for securing to the user's waist, and the second fixing means comprise a second band housed in the periphery of the end of the second receptacle, and a second tape which is a prolongation of the second band, also for securing to the user's waist.

The patent document US2012165768 discloses a urine collection device for male users with a receptacle made of impermeable material and in the form of an elongated bag for accommodation the user's penis and accumulating urine; a proximal opening located at one end of the receptacle, for introducing the penis; and a distal opening located inside the receptacle for the selective evacuation of urine, wherein the collection device additional comprises coupling means for coupling the receptacle to the patient's body.

The patent document GB994274 discloses a urine-receiver made of thin flexible polyethylene film in the form of an elongated bag.

The patent document US4673401 discloses a male incontinence device, including a urine collection receptacle which is adapted to be detachably and anchored to the leg of the patient.

The patent document GB2206050 discloses a male incontinence device including a pubic pressure plate component, an interconnector and a drainage component.

The patent document US3526227 discloses a urinal pouch having a resilient support of sheet surgical rubber with a central aperture, a pouch having a manual lower vale with its upper open end annularly bounded around the support aperture, a tubular conical resilient open ended sheath extending form the open end of the pouch and part way thereinto.

### DISCLOSURE OF THE INVENTION

The invention proposes a urine collector device according to claim 1. Further embodiments are provided in the dependent claims. The urine collector device, intended specifically for use by men, is essentially configured from a receptacle made of an impermeable material and in the form of an elongated bag for housing the penis through its proximal opening, and has a distal opening through which urine is discharged as it is connected to a draining conduit susceptible to incorporating a nozzle for the coupling thereof to a urine collection bag.

Furthermore and in order to achieve a suitable leak-free fixing to the patient's body, there is contemplated in the proximal opening thereof the existence of three coupling parts that are coupled to one another and confer the device with leak-tightness and robustness, with securing means integrated in said coupling parts.

Specifically, the collector contemplates a first coupling part, which is made of a soft and deformable material, such as anti-allergic silicone, to be fitted directly to the patient's body, on the base of the penis. The first coupling part comprises a first through hole for housing the base of the penis. The first coupling part preferably has an annular configuration.

There is coupled to this first coupling part a second coupling part or central part, also provided in turn with a second through hole in correspondence with the first through hole, where the second coupling part is preferably in the form of an essentially circular trough with a through hole and made of a material that is stiffer than the first coupling part. There can be arranged in the second through hole a membrane defined by a film made of a polymeric material which at least partially covers the second through hole and has a first opening in the central region thereof to allow the penis to go through same, providing a leak-tight communication between the second coupling part and a third coupling part that will be described below.

Securing means are also envisaged for securing an assembly formed by the receptacle plus the three coupling parts to the body of a user, where respective grooves are contemplated at opposite points of the second coupling part for the coupling of respective elongated parts or bands to which the securing means are in turn coupled, consisting preferably of belt segments that are long enough to be tied around the patient's waist and to hold the scrotum, as will be explained in further detail in a preferred embodiment.

Likewise, there is coupled to the second coupling part a third coupling part also made of a material that is stiffer than the first coupling part, where the proximal opening of the receptacle is attached thereto in a leak-tight manner preferably by means of a form-fit closure (or by means of an elastic closure). This third coupling part has a third through hole in correspondence with the first and second through holes. Like what has been described in the second coupling part, a second membrane, in a manner similar to the first membrane, can be arranged in the third through hole of the third coupling part to provide a leak-tight communication between the third coupling part and the receptacle.

Furthermore, it is important to point out that in order to make the assembly completely leak-tight, the optional existence of respective seals between the described coupling parts has been envisaged, such that between the first coupling part and the second coupling part there is incorporated a first leak-tight seal, and between the second coupling part and the third coupling part there is included a second leak-tight seal, thereby preventing, along with the soft material of the first part that fits the patient's body, any urine loss.

The collector of the invention therefore provides the following advantages:
- Complete leak-tightness, which translates into increased safety;
- A reduction in the volume it takes up as it consists of a single container, which translates into greater comfort for the patient;
- Simplicity in terms of placement since it is tied with a belt around the waist, while the other belt holds the scrotum,
- Better use can be made of it as it can be sterilized, as occurs, for example, with the silicone teats of baby bottles.

### DESCRIPTION OF THE DRAWINGS

To complement the description that is being made and for the purpose of aiding to better understand the features of the invention, a series of drawings is attached to the present specification as an integral part thereof, in which the following has been depicted in an illustrative and non-limiting character:
Figure 1 shows an exploded perspective view of an embodiment of the urine collector device object of the invention, the main portions and the elements it comprises, as well as the configuration thereof can be seen therein.
Figure 2 shows a schematic elevational view of the image depicted in Figure 1.
Figure 3 shows a detailed view of the first coupling part, partially connected to the second coupling part.
Figure 4 shows a detailed view of the first coupling part connected to the second coupling part, from an opposite perspective.
Figure 5 shows a detailed view of the second and third coupling parts, in a separated arrangement.
Figure 6 shows a detailed view of the receptacle connected both to the second and third coupling parts.

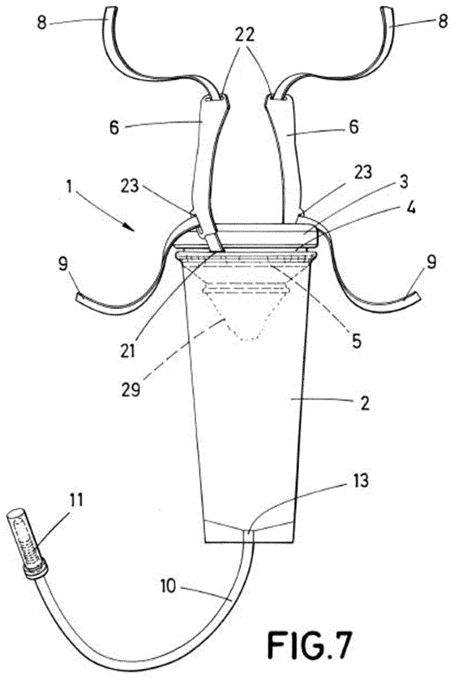

Figure 7 shows a view of the assembled device.

### DETAILED DISCLOSURE OF THE INVENTION

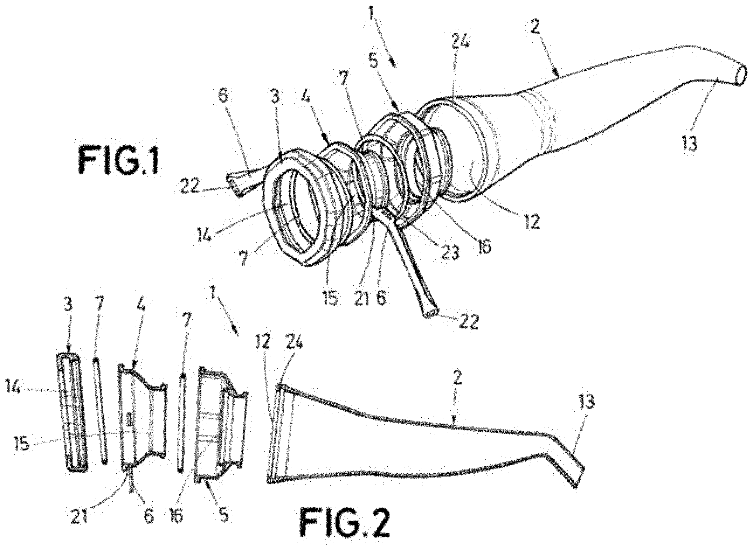

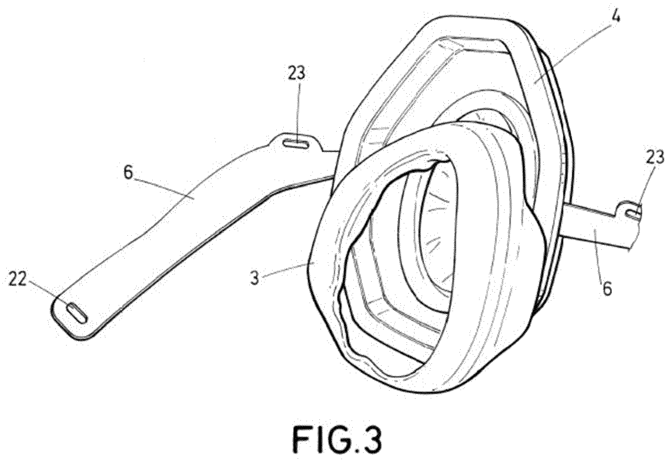

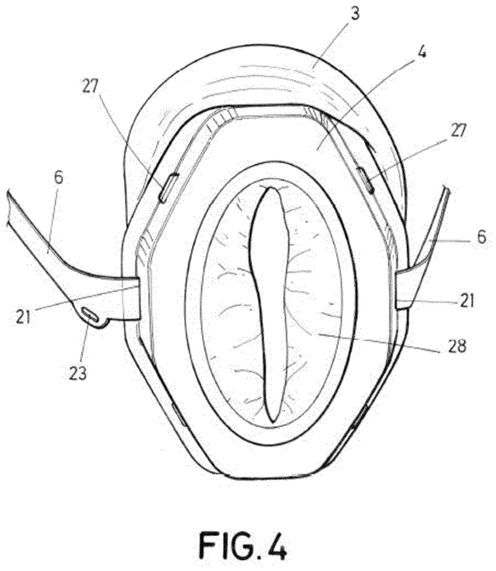

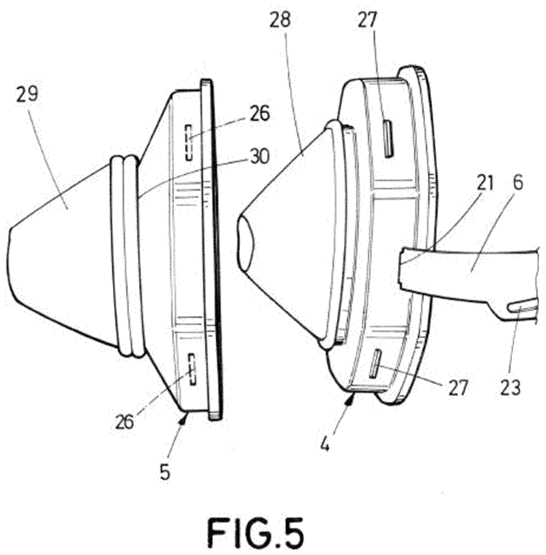

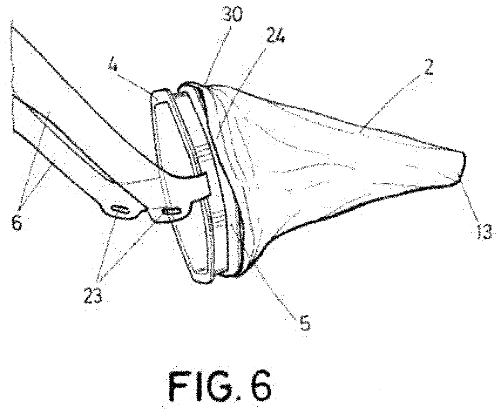

A detailed description of a preferred embodiment of the urine collector device (1) object of the present invention is provided below in reference to Figures 1-6 mentioned above.

In this sense, as seen in Figure 1 said urine collector device (1) is configured from a receptacle (2) made of an impermeable material and in the form of an elongated bag, where the receptacle (2) comprises a proximal opening (12) for housing the penis through said proximal opening (12), and it additionally comprises a distal opening (13) through which urine is discharged, where said distal opening (13) is intended for being connected to a draining conduit (10) which is in turn coupled, by means of a nozzle (11) or a valve, to a urine collection bag (not shown). The device (1) additionally comprises coupling means for coupling the receptacle (2) to the patient's body with the required safety and leak-tightness, as will be explained below. Preferably, for greater safety, the receptacle (2) is provided in the distal opening (13) with at least one check valve (not depicted) and/or a vacuum valve (also not depicted).

Additionally, in a similarly advantageous manner the receptacle (2) preferably has a length comprised between 15 cm and 25 cm. This therefore generally allows for the user's penis to be housed inside the more proximal half of the receptacle (2), so the urine that gathered therein would be allowed to take up generally the more distal half of the receptacle (2) to prevent the urine gathered therein from coming into contact with the user's penis.

To perform the aforementioned coupling, the coupling means comprise an assembly of three coupling parts (3, 4, 5) that are coupled to one another and serve to fit the receptacle (2) to the patient's body in a leak-tight manner. The coupling means further comprise securing means incorporated in at least one of the coupling parts (3, 4, 5), as will be explained below, for securing an assembly formed by the coupling parts (3, 4, 5) and the receptacle (2) to the user's body.

More specifically, the coupling parts (3, 4, 5) comprise a first coupling part (3) that is provided with an essentially annular configuration in the form of a trough, made of a soft and deformable material, for example, an elastomeric material suitable for hygienic use, such as anti-allergic silicone, for example, and comprises a first through hole (14) allowing the penis to go through same to allow the first coupling part (3) to house the base of the penis for fitting to the patient's body, the first coupling part (3) also has a proximal end intended to be arranged closer to the patient's abdomen, and a distal end, opposite the proximal end, and therefore intended for being arranged farther away from the patient's abdomen.

The coupling parts (3, 4, 5) additionally comprise a second coupling part (4) which takes up a central position in the three coupling parts (3, 4, 5) and is provided with a second through hole (15) in correspondence with the first through hole (14) of the first coupling part (3). The second coupling part (4) comprises a proximal end, closer to the patient's abdomen, and a distal end. The second coupling part (4) has an essentially annular configuration in the form of a trough, and it is stiffer than the first coupling part (3), such that the first coupling part (3) can be externally coupled, at the mentioned distal end thereof, at the proximal end of the second coupling part (4), by means of a form-fit closure due to the difference in stiffness of the materials involved. According to the example shown in the drawings, the first coupling part (3) is deformable, such that its distal end is adapted to surround the proximal end of the second coupling part (4).

The coupling parts (3, 4, 5) further comprise a third coupling part (5), also made of a material that is stiffer than the first coupling part (3), and likewise having a third through hole (16) for housing the user's penis, in correspondence with the first through hole (14) and the second through hole (15) described above. The third coupling part (5) comprises a proximal end, through which it can be connected to the second coupling part (4), and a distal end, through which it can be connected to the receptacle (2), by means of a form-fit closure. Preferably, according to the example depicted in the drawings, at the distal end of the second coupling part (4) and/or at the proximal end of the third coupling part (5) there are 10 recesses (26), as well as projections (27) that can be housed in the recesses, where the projections (27) and recesses (26) are configured to provide the mentioned form-fit closure. The drawings show, in a more specific manner, that the second coupling part (4) has projections (27) in an inner region of its distal end, whereas the third coupling part (5) has recesses (26) in an outer region of its proximal end. Other alternatives are possible, such as arranging the projections (27) in the third coupling part (4) and the recesses (26) in the second coupling part (4), or arranging both recesses (26) and projections (27), in a corresponding manner, both in the second coupling part (4) and in the third coupling part (5).

It is important to point out that the aforementioned securing means comprise attachment means (21) located in the second connecting part (4). As shown in the drawings, the attachment means (21) comprise first grooves located in said second coupling part (4), preferably at opposite points. The securing means additionally comprise respective parts in the form of bands (6) that can be connected to the attachment means (21), and said securing means also comprise two straps (8, 9) that can be connected to the bands (6), where the straps (8, 9) comprise at least a first strap (8) connected to both bands (6) to hug the user's waist, and a second strap (9) which likewise emerges from the bands (6) and is intended for limiting the movement of the user's scrotum to prevent it from hitting the receptacle (2). The scrotum is therefore confined behind the second strap (9), between said second strap (9) and the inner face of the user's thighs.

The first strap or straps (8) can be two first straps (8), each of which is connected to one of the bands (6), or can be a single first strap (8) connected to the two bands (6). The same applies for the second strap or straps (9), although having only one second strap (9) connected to both bands (6) is preferable. Preferably, as shown in the drawings the first strap or straps (8) is/are connected to the bands (6) through second grooves (22) located in the bands (6), and the second straps (9) are also preferably connected to the bands (6) through third grooves (23) located in the bands (6).

Furthermore, the existence of respective seals (7) incorporated in the coupling to prevent urine from leaking out has been envisaged. Preferably, one of the seals (7) can be arranged between the first coupling part (3) and the second coupling part (4), and the other one between the second coupling part (4) and the third coupling part (5).

The device (1) is therefore placed on the patient in the following manner:
First, the user inserts the first coupling part (3) up to the base of his penis, close to the abdomen, preferably touching the abdomen. Next, he places one of the seals (7) and then the second coupling part (4), with the first coupling part (3) surrounding said second coupling part (4), as indicated above.

Another one of the seals (7) is then assembled, with the penis inserted therethrough, and the third coupling part (5) which is connected to the second coupling part (4) according to the form-fit closure described above, is then inserted.

As indicated above, the receptacle (2) can be coupled in the proximal opening (12) to the third coupling part (5) according to a form-fit closure. By way of preferred example as depicted in the drawings, the receptacle (2) has in said proximal opening (12) an outwardly-protruding deformable perimetral thickening (24). The third coupling part (5) in turn has an outer perimetral housing (30) at the distal end, in correspondence with the perimetral thickening (24) of the receptacle (2).

The user then inserts his penis through the proximal opening (12) of the receptacle (2), and fits said receptacle (2) in the third coupling part (5) by deforming the perimetral thickening (24) and assembling it on the outer perimetral housing (30) of the third part (5), such that the assembly can withstand any pulling (caused by the weight of the urine as it gathers therein).

To improve leak-tightness and to secure the second coupling part (4) and the third coupling part (5), respective membranes (28, 29), preferably leak-tight membranes, configured by way of films made of a polymeric material, can be additionally incorporated located at the corresponding distal ends of the second coupling part (4) and the third coupling part (5). The membranes (28, 29) are preferably elastic and deformable and define impermeable conduits made of a flexible material, communicating in a leak-tight manner the second coupling part (4) with the third coupling part (5) and the third coupling part (5) with the receptacle (2), respectively. Likewise, the membranes (28, 29) at least partially cover the second through hole (15) and the third through hole (16), and have respective first and second openings in the central region thereof. Particularly, a first membrane (28) configured for being partially housed at the proximal end of the third coupling part (5) is located at the distal end of the second coupling part (4) such that the first membrane (28) at least partially covers the second through hole (15) located in the second coupling part (4) for housing the base of the penis, which first membrane (28) likewise, as indicated above, has a first opening intended to allow the penis to go through same; and a second membrane (29) configured for being partially housed at the proximal end of the receptacle (2) is additionally located at the distal end of the third coupling part (5) such that the second membrane (29) at least partially covers the third through hole (16) located in the third coupling part (5) in correspondence with the second through hole (15), where the second membrane (29) has a second opening intended to allow the penis to go through same.

## Claims

1. A urine collector device (1) for male users, comprising:
- a receptacle (2) made of an impermeable material and in the form of an elongated bag for housing the user's penis and gathering urine;
- a proximal opening (12) located at one end of the receptacle (2) into which the penis is inserted; and
- a distal opening (13) located in the receptacle (2) through which urine is discharged selectively;
the collector device (1) additionally comprises coupling means for coupling the receptacle (2) to the patient's body; wherein the coupling means comprise:
- a first coupling part (3) made of a soft and deformable material, comprising a proximal end intended to be arranged closer to the patient's abdomen, and a distal end;
- a first through hole (14) located in the first coupling part (3) for housing the base of the penis;
- a second coupling part (4) with a proximal end and a distal end;
- a second through hole (15) located in the second coupling part (4) in correspondence with the first through hole (14), where the first coupling part (3) is less stiff than the second coupling part (4), wherein the distal end of the first coupling part can be coupled at the proximal end of the second coupling part by means of a form-fit closure in a leak-tight manner due to the difference in stiffness;
- a third coupling part (5) that is made of a material that is stiffer than the first coupling part (3) and can be connected to the second coupling part (4) and to the receptacle (2) by means of respective form-fit closures;
- a third through hole (16) located in the third coupling part (5) in correspondence with the first through hole (14) and the second through hole (15); and
- securing means for attaching the coupling and the receptacle (2) to the user's body, wherein
the collector device (1) additionally comprises at least one deformable elastic membrane (28, 29) located at the corresponding distal ends of the second coupling part (4) and the third coupling part (5) for improving leak-tightness, wherein the proximal end of the third coupling part can be coupled at the distal end of the second coupling part by means of a form-fit closure as well as securing the second coupling part (4) and the third coupling part (5); where the membranes (28, 29) comprise a first membrane (28) at the distal end of the second coupling part (4), configured such that the first membrane (28) at least partially covers the second through hole (15) located in the second coupling part (4) for housing the base of the penis; the first membrane (28) likewise having a first opening intended to allow the penis to go through same and where the membranes (28, 29) comprise a second membrane (29) at the distal end of the third coupling part (5), configured such that the second membrane (29) at least partially covers the third through hole (16) located in the third coupling part (5) in correspondence with the second through hole (15); where the second membrane (29) has a second opening intended to allow the penis to go through same.

2. The urine collector device (1) according to claim 1, wherein at the distal end of the second coupling part (4) and/or at the proximal end of the third coupling part (5) there are recesses (26), as well as corresponding projections (27) that can be housed in the recesses, the projections (27) and the recesses (26) being configured to provide the mentioned form-fit closure.

3. The urine collector device (1) according to claim 1, wherein the securing means comprise:
attachment means (21) located in the second coupling part (4); two respective bands (6) that can be connected to the attachment means (21), and
at least a first strap (8) connected to both bands (6) to hug the user's waist.

4. The urine collector device (1) according to claim 3, wherein the attachment means (21) comprise first grooves located in the second coupling part (4).

5. The urine collector device (1) according to any one of claims 3 and 4, further comprises a single first strap (8) connected to the two bands (6).

6. The urine collector device (1) according to claim 3, further comprises two first straps (8) each of which is connected to one of the bands (6).

7. The urine collector device (1) according to any one of claims 3, 5 and 6, wherein the first strap or straps (8) is/are connected to the bands (6) through second grooves (22) located in the bands (6).

8. The urine collector device (1) according to any one of claims 3 to 7, additionally comprises at least a second strap (9) that emerges from the bands (6) and is intended for limiting the movement of the user's scrotum to prevent it from hitting the receptacle (2), allowing the scrotum to be confined behind the second strap (9), between said second strap (9) and the inner face of the user's thighs.

9. The urine collector device (1) according to claim 8, further comprises a single second strap (9) connected to both bands (6).

10. The urine collector device (1) according to any one of claims 8 and 9, wherein the second strap or straps (9) is/are connected to the bands (6) through third grooves (23) located in the bands (6).

11. The urine collector device (1) according to claim 1, wherein the receptacle (2) has in the proximal opening (12) an outwardly-protruding deformable perimetral thickening (24), and the third coupling part (5) has an outer perimetral housing (30) at the distal end, in correspondence with the perimetral thickening (24) of the receptacle (2) for fitting the receptacle (2) onto the third coupling part (5) by deforming the perimetral thickening (24) and assembling it on the outer perimetral housing (30) of the third part (5), such that the assembly can withstand any pulling caused by the weight of the urine as it gathers therein.

12. The urine collector device (1) according to claim 1, additionally comprises at least one seal (7) to prevent losses, which is located between the first coupling part (3) and the second coupling part (4) and/or between the second coupling part (4) and the third coupling part (5).

13. The urine collector device (1) according to claim 1, wherein the receptacle (2) is provided with a check valve in the distal opening (13).

14. The urine collector device (1) according to claim 1, wherein the receptacle (2) has a length comprised between 15 cm and 25 cm.

15. The urine collector device (1) according to any one of the preceding claims, wherein the receptacle (2) comprises a wall made of an impermeable material the thickness of which is variable.

16. The urine collector device (1) according to any one of the preceding claims, wherein the receptacle (2) comprises a reinforced thickness at the proximal end thereof in a segment of about 3 cm for reinforcing said region suitable for receiving a nozzle.

17. The urine collector device (1) according to any one of the preceding claims, wherein the receptacle (2) comprises a reinforced thickness in the proximal opening (12) for reinforcing said region.

## Patentansprüche

1. Urinsammlervorrichtung (1) für männliche Benutzer, umfassend:
- ein Behältnis (2) hergestellt aus einem undurchlässigen Material und in Form eines länglichen Beutels zur Aufnahme des Penis des Benutzers und zum Sammeln von Urin;
- eine proximale Öffnung (12), welche sich an einem Ende des Behältnisses (2) befindet und in welche der Penis eingeführt wird; und
- eine distale Öffnung (13), welche sich im Behältnis (2) befindet und durch welche Urin selektiv abgeführt wird;
wobei die Sammlervorrichtung (1) zusätzlich Kopplungsmittel zum Koppeln des Behältnisses (2) am Körper des Patienten umfasst; wobei die Kopplungsmittel Folgendes umfassen:
- einen ersten Kopplungsteil (3) hergestellt aus einem weichen und verformbaren Material, umfassend ein proximales End, welches dazu bestimmt ist, näher am Bauch des Patienten angeordnet zu werden, und ein distales End;
- ein erstes Durchgangsloch (14), welches sich im ersten Kopplungsteil (3) befindet, zur Aufnahme der Basis des Penis;
- einen zweiten Kopplungsteil (4) mit einem proximalen Ende und einem distalen Ende;
- ein zweites Durchgangsloch (15), welches sich im zweiten Kopplungsteil (4) in Übereinstimmung mit dem ersten Durchgangsloch (14) befindet, wobei der erste Kopplungsteil (3) weniger starr als der zweite Kopplungsteil (4) ist, wobei das distale Ende des ersten Kopplungsteils am proximalen Ende des zweiten Kopplungsteils mittels eines formschlüssigen Verschlusses leckdicht aufgrund vom Unterschied in Starrheit gekoppelt werden kann;
- einen dritten Kopplungsteil (5), welches aus einem Material hergestellt, welches starrer als der erste Kopplungsteil (3) ist und mit dem zweitem Kopplungsteil (4) und dem Behältnis (2) mittels jeweiliger formschlüssigen Verschlüsse verbunden werden kann;
- ein drittes Durchgangsloch (16), welches sich im dritten Kopplungsteil (5) in Übereinstimmung mit dem ersten Durchgangsloch (14) und dem zweiten Durchgangsloch (15) befindet; und
- Sicherungsmittel zum Befestigen der Kopplung und des Behältnisses (2) am Körper des Benutzers, wobei
die Sammlervorrichtung (1) zusätzlich mindestens eine verformbare elastische Membran (28, 29) umfasst, welche sich an den entsprechenden distalen Enden des zweiten Kopplungsteils (4) und des dritten Kopplungsteils (5) zum Verbessern der Leckdichtigkeit befindet, wobei das proximale Ende des dritten Kopplungsteils am distalen Ende des zweiten Kopplungsteils mittels eines formschlüssigen Verschlusses sowie unter Sicherung des zweiten Kopplungsteils (4) und des dritten Kopplungsteils (5) gekoppelt werden kann; wobei die Membranen (28, 29) eine erste Membran (28) am distalen Ende des zweiten Kopplungsteils (4) umfassen, welche derart ausgebildet ist, dass die erste Membran (28) mindestens teilweise das zweite Durchgangsloch (15) bedeckt, welches sich im zweiten Kopplungsteil (4) zur Aufnahme der Basis des Penis befindet; wobei die erste Membran (28) ebenfalls eine erste Öffnung aufweist, welche dazu bestimmt ist, den Penis zu erlauben durch dieselbe durchzugehen, und wobei die Membranen (28, 29) eine zweite Membran (29) am distalen Ende des dritten Kopplungsteils (5) umfassen, welche derart ausgebildet ist, dass die zweite Membran (29) mindestens teilweise das dritte Durchgangsloch (16) bedeckt, welches sich im dritten Kopplungsteil (5) in Übereinstimmung mit dem zweiten Durchgangsloch (15) befindet; wobei die zweite Membran (29) eine zweite Öffnung aufweist, welche dazu bestimmt ist, den Penis zu erlauben durch dieselbe durchzugehen.

2. Urinsammlervorrichtung (1) nach Anspruch 1, wobei es am distalen Ende des zweiten Kopplungsteils (4) und/oder am proximalen Ende des dritten Kopplungsteils (5) Aussparungen (26) sowie entsprechende Vorsprünge (27), welche in die Aussparungen aufgenommen werden können, gibt, wobei die Vorsprünge (27) und die Aussparungen (26) dazu ausgebildet sind, den erwähnten formschlüssigen Verschluss bereitzustellen.

3. Urinsammlervorrichtung (1) nach Anspruch 1, wobei die Sicherungsmittel Folgendes umfassen:
Befestigungsmittel (21), welche sich im zweiten Kopplungsteil (4) befinden;
zwei jeweilige Bänder (6), welche mit den Befestigungsmittel (21) verbunden werden können, und
mindestens einen ersten Riemen (8), welcher mit beiden Bänder (6) verbunden ist, um die Taille des Benutzers zu umklammern.

4. Urinsammlervorrichtung (1) nach Anspruch 3, wobei die Befestigungsmittel (21) erste Rillen umfassen, welche sich im zweiten Kopplungsteil (4) befinden.

5. Urinsammlervorrichtung (1) nach einem der Ansprüche 3 und 4, zusätzlich umfassend einen einzigen ersten Riemen (8), welche mit den zwei Bändern (6) verbunden ist.

6. Urinsammlervorrichtung (1) nach Anspruch 3, zusätzlich umfassend zwei erste Riemen (8), welche jeweils mit einem der Bänder (6) verbunden sind.

7. Urinsammlervorrichtung (1) nach einem der Ansprüche 3, 5 und 6, wobei der erste oder die ersten Riemen (8) mit den Bändern (6) über zweite Rillen (22) verbunden ist/sind, welche sich in den Bändern (6) befinden.

8. Urinsammlervorrichtung (1) nach einem der Ansprüche 3 bis 7, zusätzlich umfassend mindestens einen zweiten Riemen (9), welcher aus den Bändern (6) hervorgeht und dazu bestimmt ist, die Bewegung des Hodensacks des Benutzers zu begrenzen, um zu verhindern, dass es gegen das Behältnis (2) stößt, so dass es erlaubt wird, dass der Hodensack hinter dem zweiten Riemen (9), zwischen dem genannten zweiten Riemen (9) und der inneren Fläche der Oberschenkel des Benutzers, abgegrenzt wird.

9. Urinsammlervorrichtung (1) nach Anspruch 8, zusätzlich umfassend einen einzigen zweiten Riemen (9), welcher mit beiden Bändern (6) verbunden ist.

10. Urinsammlervorrichtung (1) nach einem der Ansprüche 8 und 9, wobei der zweite oder die zweiten Riemen (9) mit den Bändern (6) über dritte Rillen (23) verbunden ist/sind, welche sich in den Bändern (6) befinden.

11. Urinsammlervorrichtung (1) nach Anspruch 1, wobei das Behältnis (2) in der proximalen Öffnung (12) eine nach außen hervorstehende verformbare Umfangsverdickung (24) aufweist, und der dritte Kopplungsteil (5) ein äußeres Umfangsgehäuse (30) am distalen Ende, in Übereinstimmung mit der Umfangsverdickung (24) des Behältnisses (2) aufweist, zum Einpassen des Behältnisses (2) auf dem dritten Kopplungsteil (5) mittels der Verformung der Umfangsverdickung (24) und zum Montieren desselben auf dem äußeren Umfangsgehäuse (30) des dritten Teils (5), so dass die Baueinheit jeden Zug standhalten kann, welcher vom Gewicht des Urins hervorgerufen wird, während es sich darin sammelt.

12. Urinsammlervorrichtung (1) nach Anspruch 1, zusätzlich umfassend mindestens eine Dichtung (7), um Verluste zu verhindern, welche sich zwischen dem ersten Kopplungsteil (3) und dem zweiten Kopplungsteil (4) und/oder zwischen dem zweiten Kopplungsteil (4) und dem dritten Kopplungsteil (5) befindet.

13. Urinsammlervorrichtung (1) nach Anspruch 1, wobei das Behältnis (2) mit einem Rückschlagventil in der distalen Öffnung (13) versehen ist.

14. Urinsammlervorrichtung (1) nach Anspruch 1, wobei das Behältnis (2) eine Länge aufweist, welche zwischen 15 cm und 25 cm liegt.

15. Urinsammlervorrichtung (1) nach einem der vorhergehenden Ansprüche, wobei das Behältnis (2) eine aus einem undurchlässigen Material hergestellte Wand umfasst, deren Dicke variabel ist.

16. Urinsammlervorrichtung (1) nach einem der vorhergehenden Ansprüche, wobei das Behältnis (2) eine verstärkte Dicke am proximalen Ende desselben in einem Segment von etwa 3 cm umfasst, zum Verstärken des genannten Bereichs, damit es zum Empfangen einer Düse geeignet ist.

17. Urinsammlervorrichtung (1) nach einem der vorhergehenden Ansprüche, wobei das Behältnis (2) eine verstärkte Dick in der proximalen Öffnung (12) umfasst, zum Verstärken des genannten Bereichs.

## Revendications

1. Dispositif collecteur d'urine (1) pour utilisateurs masculins comprenant:
- Un réceptacle (2) constitué d'une matière imperméable et en forme d'un sac allongé pour loger le pénis de l'utilisateur et recueillir l'urine;
- Une ouverture proximale (12) située à une extrémité du réceptacle (2) dans laquelle est inséré le pénis; et
- Une ouverture distale (13) située dans le réceptacle (2) à travers laquelle l'urine est déchargée de manière sélective;
le dispositif collecteur (1) comprend en outre des moyens de couplage pour coupler le réceptacle (2) au corps du patient; dans lequel les moyens de couplage comprennent :
- Une première partie de couplage (3) réalisée en une matière molle et déformable, comprenant une extrémité proximale destinée à être aménagée plus près de l'abdomen du patient, et une extrémité distale;
- un premier trou traversant (14) situé dans la première partie de couplage (3) pour loger la base du pénis;
- une deuxième partie de couplage (4) avec une extrémité proximale et une extrémité distale;
- un deuxième trou traversant (15) situé dans la deuxième partie de couplage (4) en correspondance avec le premier trou traversant (14), où la première partie de couplage (3) est moins rigide que la deuxième partie de couplage (4), dans lequel l'extrémité distale de la première partie de couplage peut être couplée à l'extrémité proximale de la deuxième partie de couplage par le biais d'une fermeture ergonomique d'une manière étanche aux fuites à cause de la différence de rigidité;
- une troisième partie de couplage (5) qui est réalisée en une matière qui est plus rigide que la première partie de couplage (3) et qui peut être reliée à la deuxième partie de couplage (4) et au réceptacle (2) par le biais de fermetures ergonomiques respectives;
- un troisième trou traversant (16) situé dans la troisième partie de couplage (5) en correspondance avec le premier trou traversant (14) et le deuxième trou traversant (15); et
- des moyens de fixation pour fixer le couplage et le réceptacle (2) au corps de l'utilisateur, dans lequel
le dispositif collecteur (1) comprend en outre au moins une membrane déformable élastique (28, 29) située aux extrémités distales correspondantes de la deuxième partie de couplage (4) et la troisième partie de couplage (5) pour améliorer l'étanchéité aux fuites, dans lequel l'extrémité proximale de la troisième partie de couplage peut être couplée à l'extrémité distale de la deuxième partie de couplage par le biais d'une fermeture ergonomique ainsi qu'en assurant la deuxième partie de couplage (4) et la troisième partie de couplage (5); où les membranes (28, 29) comprennent une première membrane (28) à l'extrémité distale de la deuxième partie de couplage (4), configurée de manière à ce que la première membrane (28) recouvre au moins partiellement le deuxième trou traversant (15) situé dans la deuxième partie de couplage (4) pour loger la base du pénis; la première membrane (28) ayant également une première ouverture destinée à permettre que le pénis passe à travers celle-ci et où les membranes (28, 29) comprennent une deuxième membrane (29) à l'extrémité distale de la troisième partie de couplage (5), configuré de manière que la deuxième membrane (29) recouvre au moins partiellement le troisième trou traversant (16) situé dans la troisième partie de couplage (5) en correspondance avec le deuxième trou traversant (15); où la deuxième membrane (29) a une deuxième ouverture destinée à permettre que le pénis passe à travers celle-ci.

2. Dispositif collecteur d'urine (1) selon la revendication 1, dans lequel à l'extrémité distale de la deuxième partie de couplage (4) et/ou à l'extrémité proximale de la troisième partie de couplage (5) il y a des évidements (26), ainsi que des projections correspondantes (27) qui peuvent être logées dans les évidements, les projections (27) et les évidements (26) étant configurés pour fournir ladite fermeture ergonomique.

3. Dispositif collecteur d'urine (1) selon la revendication 1, dans lequel les moyens de fixation comprennent:
moyens de retenue (21) situés dans la deuxième partie de couplage (4);
deux bandes respectives (6) qui peuvent être reliées aux moyens de retenue (21), et
au moins une première sangle (8) reliée aux deux bandes (6) pour étreindre à la taille de l'utilisateur.

4. Dispositif collecteur d'urine (1) selon la revendication 3, dans lequel les moyens de retenue (21) comprennent des premières rainures situées dans la deuxième partie de couplage (4).

5. Dispositif collecteur d'urine (1) selon l'une quelconque des revendications 3 et 4, comprend en outre une première sangle individuelle (8) reliée aux deux bandes (6).

6. Dispositif collecteur d'urine (1) selon la revendication 3, comprend en outre deux premières sangles (8), chacune desquelles est reliée à une des bandes (6).

7. Dispositif collecteur d'urine (1) selon l'une quelconque des revendications 3, 5 et 6, dans lequel la première sangle ou premières sangles (8) est/sont reliées aux bandes (6) à travers les deuxième rainures (22) situées dans les bandes (6).

8. Dispositif collecteur d'urine (1) selon l'une quelconque des revendications 3 à 7, comprend en outre au moins une deuxième sangle (9) qui émerge des bandes (6) et qui est destinée à limiter le mouvement du scrotum de l'utilisateur pour éviter de heurter le réceptacle (2), en permettant que le scrotum soit confiné derrière la deuxième sangle (9), entre ladite deuxième sangle (9) et la face intérieure des cuisses de l'utilisateur.

9. Dispositif collecteur d'urine (1) selon la revendication 8, comprend en outre une deuxième sangle individuelle (9) reliée aux deux bandes (6).

10. Dispositif collecteur d'urine (1) selon l'une quelconque des revendications 8 et 9, dans lequel la deuxième sangle ou deuxièmes sangles (9) est/sont reliées aux bandes (6) à travers des troisièmes rainures (23) situées dans les bandes (6).

11. Dispositif collecteur d'urine (1) selon la revendication 1, dans lequel le réceptacle (2) a dans l'ouverture proximale (12) une épaisseur périmétrique déformable saillante extérieurement (24), et la troisième partie de couplage (5) a un logement périmétrique extérieur (30) à l'extrémité distale, en correspondance avec l'épaisseur périmétrique (24) du réceptacle (2) pour s'ajuster au réceptacle (2) sur la troisième partie de couplage (5) en déformant l'épaisseur périmétrique (24) et en l'assemblant sur le logement périmétrique extérieur (30) de la troisième partie (5), de manière que l'assemblage puisse résister toute traction produite par le poids de l'urine contenue en son sein.

12. Dispositif collecteur d'urine (1) selon la revendication 1, comprend en outre au moins un joint (7) pour éviter les pertes, qui est situé entre la première partie de couplage (3) et la deuxième partie de couplage (4) et/ou entre la deuxième partie de couplage (4) et la troisième partie de couplage (5).

13. Dispositif collecteur d'urine (1) selon la revendication 1, dans lequel le réceptacle (2) est pourvu d'un clapet anti-retour dans l'ouverture distale (13).

14. Dispositif collecteur d'urine (1) selon la revendication 1, dans lequel le réceptacle (2) a une longueur comprise entre 15 cm et 25 cm.

15. Dispositif collecteur d'urine (1) selon l'une quelconque des revendications précédentes, dans lequel le réceptacle (2) comprend une paroi réalisée en une matière imperméable dont l'épaisseur est variable.

16. Dispositif collecteur d'urine (1) selon l'une quelconque des revendications précédentes, dans lequel le réceptacle (2) comprend une épaisseur renforcée à son extrémité proximale dans un segment d'environ 3 cm pour renforcer ladite région appropriée pour recevoir une buse.

17. Dispositif collecteur d'urine (1) selon l'une quelconque des revendications précédentes, dans lequel le réceptacle (2) comprend une épaisseur renforcée dans l'ouverture proximale (12) pour renforcer ladite région.
